# EUROPÄISCHE PATENTSCHRIFT

(11) **EP 2 380 602 B1**
(45) Veröffentlichungstag und Bekanntmachung des Hinweises auf die Patenterteilung: **22.04.2015**
(21) Anmeldenummer: 10003941.1
(22) Anmeldetag: 14.04.2010
(51) Int. Cl.: A61L 27/54, A61L 31/16, A61K 47/18, C12N 15/00

(54) **Kunststoffe enthaltend Nukleinsäuren in komplexierter Form und Verfahren zu deren Herstellung**
Nucleic acids containing plastics in complexed form and method for producing same
Matières synthétiques contenant des acides nucléiques sous forme complexe et leur procédé de fabrication

(43) Veröffentlichungstag der Anmeldung: 26.10.2011
(73) Patentinhaber: Kosak, Hans, 53115 Bonn (DE)
(72) Erfinder: Kosak, Hans, 53115 Bonn (DE)
(74) Vertreter: Ehnis, Tobias

(56) Entgegenhaltungen:
- WO-A1-01/52915
- WO-A1-2009/119499
- US-A1- 2009 186 059
- BOUSSIF O. ET AL: 'A VERSATILE VECTOR FOR GENE AND OLIGONUCLEOTIDE TRANSFER INTO CELLS IN CULTURE AND IN VIVO: POLYETHYLENIMINE' PROCEEDINGS OF THE NATIONAL ACADEMY OF SCIENCES, NATIONAL ACADEMY OF SCIENCES, US Bd. 92, 01 August 1995, Seiten 7297 - 7301, XP002947333 DOI: 10.1073/PNAS.92.16.7297 ISSN: 0027-8424

## Beschreibung

Gegenstand der vorliegenden Anmeldung sind ein Kunststoff enthaltend mindestens eine Nukleinsäure, die derart komplexiert ist, dass die negativen Ladungen der Nukleinsäure im Wesentlichen vollständig durch organische Kationen kompensiert sind, wobei die organischen Kationen quartäre Ammoniumionen umfassen und jedes organisches Kation eine kationische Gruppe trägt, sowie Verfahren zur Herstellung des Kunststoffs.

### Stand der Technik

Aus der US 2004/0058374 A1 ist eine Methode zum Mischen von Nukleinsäure mit einem wasserunlöslichen Medium bekannt, bei der Nukleinsäurelösung durch die Zugabe einer sogenannten intermediären Lösung in ein wasserunlösliches Medium eingemischt werden kann. Beispielsweise kann DNA in Wasser gelöst und mit einer Mischung aus Ethanol und Aceton als intermediärer Lösung gemischt und sodann mit organischem Lösungsmittel wie Chloroform gemischt werden. In Letzterem kann auch ein Kunststoff wie Polystyrol gelöst sein. Diese Lösung kann zum Markieren von Feststoffen oder Gegenständen verwendet werden, indem die Lösung darauf aufgetragen wird. Nachteilig an dem Verfahren der US 2004/0058374 A1 ist, dass der größte Teil der Nukleinsäure in der wässrigen Phase außerhalb des gegebenenfalls vorhandenen Kunststoffs verbleibt, d.h. in diesen nicht effizient eingebaut wird.

Die DE 696 37 171 T2 betrifft Zusammensetzungen zur Gentherapie, die mit Nukleinsäure beladene Mikropartikel aus Polymeren enthalten. Diese werden hergestellt, indem eine wässrige Nukleinsäurelösung mit einem Lösungsmittel wie Dichlormethan, das in Wasser nicht löslich ist und in dem ein Polymer gelöst ist, gemischt wird und das Wasser sodann durch Gefriertrocknung entzogen wird. Der so gebildete Nukleinsäure-Polymerkomplex wird in Lösungsmittel aufgenommen und mit einer weiteren Flüssigkeit wie Petrolether gemischt, um die gewünschten Mikropartikel zu erzeugen. Ein Nachteil dieses Verfahrens ist, dass die Nukleinsäure ungeschützt auf der Oberfläche der Mikropartikel angeordnet und damit einem enzymatischen Abbau zugänglich ist. Ein weiterer Nachteil ist, dass keine Vermittler zur Aufnahme der Mikropartikel in die Zielzellen vorhanden ist.

WO 01/52915 A1 beschreibt beschichtete Implantate, die auf ihrer äußeren Fläche eine vernetzte, in Wasser quellbare Polymermatrix und eine pharmazeutisch aktive Verbindung wie eine Nukleinsäure aufweisen, wobei das Polymer der Matrix seitenständige zwitterionische Gruppen und kationische Gruppen aufweist.

US 2009/186059 A1 betrifft ein Verfahren zur Herstellung medizinischer Produkte wie Implantate, bei dem Nukleinsäuren mit einem Träger zur Bildung einer Lösung eines Verabreichungskomplexes kontaktiert werden, der Komplex auf ein Substrat aufgebracht wird und eine polymere Lösung auf das Substrat einwirkt. Der Träger kann ein neutrales oder ein kationisches Makromolekül umfassen. Ferner kann die Polymerlösung ein Vernetzungsmittel umfassen, das positiv geladen sein kann oder ladungsneutral ist.

WO 2009/119499 A1 beschreibt einen Stent, dessen Oberfläche mit einem Komplex beschichtet ist, der eine Nukleinsäure enthält, die ecto-ATPDase codiert. Der Komplex enthält ferner ein positiv geladenes wasserunlösliches und biologisch abbaubares Polymer.

Aufgabe der vorliegenden Erfindung ist es Kunststoffe mit darin enthaltenen Nukleinsäuren zur Verfügung zu stellen, die für eine Vielzahl von Anwendungen geeignet sind und auf effiziente Weise herstellbar sind, wobei insbesondere die Nachteile des Standes der Technik vermieden werden können. Insbesondere ist es eine Aufgabe der vorliegenden Erfindung, Kunststoffe mit darin enthaltenen Nukleinsäuren zur Verfügung zu stellen, die als fälschungssichere Markierung und/oder zur Kontrolle der Verarbeitungswege der Kunststoffe dienen können. Eine weitere Aufgabe der vorliegenden Erfindung ist es, Kunststoffe mit darin enthaltenen Nukleinsäuren zur Verfügung zu stellen, die für medizinische und pharmazeutische Anwendungen geeignet sind, oder die zu therapeutischen Zwecken mit Zellen oder Gewebe von Mensch und Tier in Kontakt gebracht werden können.

### Zusammenfassung der Erfindung

Die vorliegende Erfindung betrifft einen Kunststoff, der mindestens eine Nukleinsäure enthält und dadurch gekennzeichnet ist, dass die Nukleinsäure derart komplexiert ist, dass die negativen Ladungen der Nukleinsäure durch organische Kationen im Wesentlichen vollständig kompensiert sind, wobei die organischen Kationen quartäre Ammoniumionen umfassen und jedes organische Kation eine kationische Gruppe trägt.

Ferner betrifft die vorliegende Erfindung Verfahren zur Herstellung des obigen Kunststoffs, bei dem
a) zumindest die folgenden Bestandteile:
   i) mindestens eine komplexierte Nukleinsäure, deren negative Ladungen durch organische Kationen im Wesentlichen vollständig komplexiert sind,
   ii) mindestens ein polymerer Kunststoff oder ein oder mehrerere verschiedene Monomere, aus denen der polymere Kunststoff gebildet wird,
   iii) gegebenenfalls organisches Lösungsmittel, in dem die komplexierte Nukleinsäure und der polymere Kunststoff löslich sind, soweit die Monomere nicht bereits ein Lösungsmittel für die komplexierte Nukleinsäure darstellen,
   gleichzeitig oder nacheinander zu einer Flüssigkeit vermischt werden,
b) das gegebenenfalls vorhandene Lösungsmittel anschließend abgetrennt wird bzw. die Polymerisation der Monomere durchgeführt wird, wodurch ein Kunststoff entsteht, in dem die negativen Ladungen der Nukleinsäure durch organische Kationen im Wesentlichen vollständig komplexiert sind.

Bevorzugte Ausführungsformen sind in der nachfolgenden Beschreibung und den abhängigen Ansprüchen offenbart.

### Detaillierte Beschreibung der Erfindung

Erfindungsgemäß wird unter Kunststoff ein organisches Homopolymer oder Copolymer oder höheres Copolymer (z.B. ein Terpolymer) verstanden, das normalerweise als Festkörper (bei Standardbedingungen) vorliegt und dessen Grundgerüst synthetisch oder halbsynthetisch aus monomeren organischen Molekülen hergestellt wird. Mit Kunststoff werden vorliegend auch solche Polymere bezeichnet, die aus natürlichen Quellen stammen, aber chemisch-synthetisch, gegebenenfalls auch nur geringfügig modifiziert worden sind. Von Bedeutung für die erfindungsgemäße Verwendbarkeit der Polymere ist, dass sie geeignete Lösbarkeit zusammen mit den Nukleinsäuren aufweisen bzw. dass die ihnen zu Grunde liegenden Monomere als "Lösungsmittel" dienen können.

Unter einer geladenen Nukleinsäure ist erfindungsgemäß eine Nukleinsäure zu verstehen, deren Nukleotide durch Phosphordiesterbindungen miteinander verknüpft sind, wobei die an den Phosphordiesterbindungen beteiligten Phosphatreste negativ geladen sind, wie es bei natürlich verkommender DNA oder RNA der Fall ist.

Mit anderen Worten handelt es sich bei den geladenen Nukleinsäuren um polyanionische Säuren (Polyelektrolyte), in denen die anionischen Gruppen durch die negativ geladenen Sauerstoffreste der Phosphatgruppen gebildet werden, die die Phosphordiestherbindungen bilden. Grundsätzlich kann es sich bei den die negativen Ladungen tragenden Resten der Nukleinsäuren auch um die negativen Reste von Phosphorthioaten oder Phosphordithioaten oder auch um andere negativ geladene Gruppen von Nukleinsäuren handeln.

Üblicherweise lösen sich Nukleinsäuren auf Grund ihrer Ladung gut in Wasser, nicht jedoch in mit Wasser nicht mischbaren organischen Flüssigkeiten, wie Kohlenwasserstoffen oder anderen organischen Lösungsmitteln wie Chloroform etc. Eine Nukleinsäure wird im Sinne der Erfindung als gelöst erachtet, wenn sie sich durch eine 5-minütige Zentrifugation bei 15000 x g nicht abzentrifugieren lässt.

Bevorzugt sind die Nukleinsäuren synthetisch hergestellte Nukleinsäuren mit bekannter Sequenz. Dadurch lässt sich eine nahezu unbegrenzte Zahl von Codierungen zur fälschungssicheren Markierung bereitstellen. Besonders bevorzugt ist es, wenn dazu die zur Identifizierung verwendeten Nukleinsäuren in einer großen Zahl weiterer in der ersten Flüssigkeit gelöster Nukleinsäuren "versteckt" werden, so dass durch eine Sequenzierung nicht die spezifisch zur Markierung dienenden Nukleinsäuren ermittelt werden können. Zur Bereitstellung der weiteren Nukleinsäuren kann z.B. Heringsperm-DNA verwendet werden.

Die Nukleinsäuren können jeweils eine Kettenlänge von 5 bis 100 Nukleotiden, bevorzugt 10 bis 80 Nukleotiden, besonders bevorzugt 15 bis 60 Nukleotiden, aufweisen. Bei den Nukleinsäuren kann es sich um DNA, insbesondere Antisinn-DNA, oder RNA, insbesondere siRNA, handeln. Für eine therapeutische Anwendung ist es besonders vorteilhaft, wenn die Nukleinsäuren zumindest einen Teil einer Sequenz eines humanen oder tierischen Gens enthalten.

Die Nukleinsäuren können einzelsträngig oder doppelsträngig ausgebildet sein. Eine doppelsträngig ausgebildete Nukleinsäure kann in Form zweier separater Einzelstränge oder als Haarnadelschleifenstruktur vorliegen.

Erfindungsgemäß werden die negativen Ladungen der Nukleinsäure im Wesentlichen vollständig durch organische Kationen kompensiert. Dabei liegt vorzugsweise ein stöchiometrisches (Mol-)Verhältnis von 1:1 zwischen den negativen Ladungen, d.h. anionischen Gruppen, der Nukleinsäure und den Ladungen der organischen Kationen vor. Insbesondere trägt jedes organische Kation eine positive Ladung, sodass vorzugsweise das stöchiometrische Verhältnis zwischen den organischen Kationen und den negativen Ladungen (anionischen Gruppen) der Nukleinsäure 1:1 beträgt.

Mit anderen Worten, kann der Komplex aus Nukleinsäure und organischen Kationen so eingestellt sein, dass im Wesentlichen kein Überschuss an Kationen vorhanden ist. Dadurch sind die Komplexe aus Nukleinsäuren und organischen Kationen im Wesentlichen nach außen ungeladen.

Vorteilhaft an der Einstellung eines solchen Verhältnisses ist, dass bei Kontakt der so komplexierten Nukleinsäure mit einer lebenden Zelle oder einem Organ eines Lebewesens keine bzw. nur wenige freie organische Kationen mit den Zellen oder dem Organismus wechselwirken können. Als Folge davon können die organischen Kationen keine cytotoxischen Effekte bei den Zellen auslösen, was ansonsten häufig vorkommen kann.

Ein weiterer Vorteil der Verwendung eines weitgehend stöchiometrischen Verhältnisses zwischen den organischen Kationen und den negativen Ladungen der Nukleinsäure ist, dass der Komplex aus organischen Kationen und Nukleinsäure insgesamt weitgehend ladungsneutral ist. Dies hat zum Einen zur Folge, dass die komplexierte Nukleinsäure in einem wässrigen Medium unlöslich wird, bei der Herstellung in einem wässrigen Medium unlöslich wird, bei der Herstellung in wässrigen Medien als unlösliches Präzipitat ausfällt und so leichter abgetrennt werden kann.

Diese Eigenschaft erleichtert also die Herstellung der gewünschten, stöchiometrisch komplexierten Nukleinsäure.

Ein weiterer Vorteil der Einstellung einer stöchiometrischen Komplexierung ist die resultierende Löslichkeit in einer Vielzahl von organischen Lösungsmitteln einschließlich solchen, die allgemein als Fällungsmittel für Nukleinsäure aus wässriger Lösung gelten. Dies betrifft insbesondere die Verwendung von niederen Alkoholen wie Ethanol, Propanol oder Butanol. In derartigen Lösungsmitteln kann sogar eine Konzentration an komplexierter Nukleinsäure erreicht werden wie in neutralen oder wässrigen Medien, d.h. Konzentrationen von mehr als 10 µg Nukleinsäure pro µl Lösungsmittel können erreicht werden, zum Beispiel in Butanol oder Ethylenglykolethylether (EGE).

Ein weiterer Vorteil der Einarbeitung von Nukleinsäuren in organische Lösungsmittel ist die daraus resultierende Stabilität. Dies ist im Wesentlichen darauf zurückzuführen, dass Nukleasen in nicht-wässriger Umgebung inaktiv sind. Dies ist vor allem bei Verwendung von Ribonukleinsäure (RNA) als Nukleinsäure von Bedeutung, da in allen wässrigen Systemen mit einer hohen RNAse-Aktivität gerechnet werden muss. Außerhalb einer speziellen, RNAse-freien Umgebung, die praktisch nur unter speziellen Laborbedingungen realisiert werden kann, ist ansonsten mit einem schnellen Abbau der RNA zu rechnen, was insbesondere den Einsatz von RNA als Pharmakon erschwert. Dieses Problem wird durch die bei der vorliegenden Erfindung verwendete Komplexierung mit anschließendem Einbau in einen Kunststoff gelöst.

Ferner ist die Stabilität der komplexierten, gelösten Nukleinsäuren auch dadurch erhöht, dass in dem eingesetzten organischen Lösungsmittel bzw. Kunststoff keine saure oder alkalische Umgebung mehr vorliegt. Dies vermeidet eine etwaige saure oder alkalische Hydrolyse der Nukleinsäurestrukturen.

Schließlich wirkt sich auf die Stabilität der Nukleinsäure vorteilhaft aus, dass die optischen Eigenschaften des Lösungsmittels bzw. des Kunststoffs durch eine vergleichsweise höhere UV-Strahlungsabsorption gekennzeichnet sind, verglichen mit Wasser. Dadurch wird die bei Verwendung von wässrigen Medien ansonsten mögliche UV-Schädigung der Nukleinsäurestrukturen vermindert bzw. vollständig vermieden. Zum Beispiel führt die Verwendung von Kunststoffen, die aromatische Strukturen enthalten, die bei einer Wellenlänge von um 260 nm absorbieren, zu einer weitestgehenden Abschirmung der für die Nukleinsäure schädigenden UV-Strahlung.

Ein Merkmal der erfindungsgemäßen Komplexierung der Nukleinsäuren ist die räumliche Anordnung der organischen Kationen in der komplexierten Nukleinsäure. Die verwendeten organischen Kationen wie insbesondere CTA sind in der Lage, die positiv geladene Gruppe in einen ausreichend kleinen Abstand an das negativ geladene Sauerstoffatom der Nukleinsäure heranzubringen, sodass eine ausreichende (lokale) Abschirmung der Gesamtladung beider Ionen bewirkt wird.

Bei der Herstellung des erfindungsgemäßen polymeren Kunststoffs wird im Allgemeinen zunächst die komplexierte Nukleinsäure hergestellt. Dabei kann so vorgegangen werden, wie in der DE 10 2006 038 791 offenbart. Dabei werden eine oder mehrere Nukleinsäuren wie vorstehend beschrieben in wässrigem Medium, insbesondere demineralisiertes Wasser, mit einer geeigneten Quelle für die organischen Kationen in Kontakt gebracht, die sodann zur Komplexierung dienen. Geeignete Quellen für organische Kationen sind kationische Detergenzien, Lipide und Stickstoffverbindungen mit quartärem Stickstoff wie in organischen Ammoniumsalzen.

Erfindungsgemäß wird als Quelle für organische Kationen eine oder mehrere Verbindungen der Formel NR₄X verwendet, wobei R jeweils unabhängig voneinander ein Kohlenwasserstoffrest mit 1 bis 20 Kohlenstoffatomen, der verzweigt oder unverzweigt sein kann, ist und X ein Halogen ausgewählt aus der Gruppe bestehend aus Chlor, Brom, Iod ist, insbesondere Chlor oder Brom, vorzugsweise Brom. Der Kohlenstoffrest kann auch ein oder mehrere Heteroatome enthalten soweit das nicht seinen überwiegenden Kohlenwasserstoffcharakter signifikant ändert. Insbesondere enthält der Kohlenwasserstoffrest jedoch nur C- und H-Atome.

Gemäß weiteren Ausführungsformen der Erfindung kann die Quelle für organische Kationen eine oder mehrere Verbindungen der Formel NR¹R²R³R⁴X sein, wobei R¹, R², R³ und R⁴ organische Reste darstellen, insbesondere Kohlenwasserstoffreste wie vorstehend definiert, und wobei zumindest R¹ ein C1-Rest ist und R² bis R⁴ längerkettige Reste sind oder
R¹ und R² jeweils ein C1-Rest sind und R³ sowie R⁴ längerkettige Reste sind oder
R¹, R² und R³ jeweils ein C1-Rest sind und R⁴ ein längerkettiger Rest ist.

Insbesondere sind die Reste R¹, R², R³ und R⁴ wie nachstehend definiert, wobei der Kürze halber nur auf "R" Bezug genommen wird.

R ist in den Verbindungen der Formel NR₄X vorzugsweise ein Alkylrest mit 1 bis 20 Kohlenstoffatomen, der verzweigt oder unverzweigt sein kann. Insbesondere können einer oder mehrere, insbesondere zwei oder drei, von R relativ kurzkettige Reste wie C1 bis C3, wie Methyl, Ethyl und Propyl sein, während ein R ein längerer Rest wie C8 oder C10 bis C20, insbesondere C10 bis C16, wie Octyl, Nonyl, Decyl, Undecyl, Dodecyl, Tridecyl, Tetradecyl, Pentadecyl, Cetyl, Heptadecyl, Stearyl, Nonadecyl oder Icosanyl ist. Eine bevorzugte Kombination von Resten R ist drei Methylreste und ein längerkettiger Rest, insbesondere ein C10 bis C20-Kohlenwasserstoffrest wie vorstehend definiert wie insbesondere ein Cetylrest wie in Cetyltrimethylammoniumbromid (CTAB).

Nach Kontaktierung mit der Quelle für organische Kationen zur Komplexierung der Nukleinsäure bildet sich ein Niederschlag, der geeigneterweise durch Zentrifugation, zum Beispiel für eine Zeit von 1 bis 10 Minuten, wie 5 Minuten, bei 10000 bis 20000 Upm, wie 15000 Upm abgetrennt werden kann. Gegebenenfalls kann dieser Vorgang nach erneuter Aufnahme des gefällten Niederschlags in Wasser mehrmals durchgeführt werden.

Sodann wird der abgetrennte Niederschlag aus komplexierter Nukleinsäure in einem geeigneten organischen Lösungsmittel aufgenommen. Als organische Lösungsmittel sind insbesondere ein- oder mehrwertige Alkohole oder teilweise veretherte Alkohole geeignet. Insbesondere sind C₁- bis C₈-Alkohole geeignet wie Methanol, Ethanol, 1-Propanol oder 2-Propanol, Butanol, wie 1-Butanol, Pentanol wie 1-Pentanol oder Pentandiol wie 1,5-Pentandiol, Hexanol, Heptanol und Octanol. Ferner sind zweiwertige Alkohole wie Ethylenglykol oder teilweise veretherte Derivate davon geeignet wie Ethylenglykol, das mit Methanol, Ethanol, Propanol oder Butanol verethert ist, wie beispielsweise Ethylenglykolether wie insbesondere Ethylenglykolmonobutylether oder -ethylether, oder dreiwertige Alkohole wie Glycerin. Alternativ können gesättigte oder ungesättigte paraffinische Kohlenwasserstoffe wie Pentan, Hexan oder Heptan, cyclische Kohlenwasserstoffe wie Cyclohexan oder auf Benzol basierende aromatische Kohlenwasserstoffe wie Xylol oder Styrol verwendet werden, also insbesondere mit einem oder mehreren C1 bis C3-Resten substituierte Benzole. Alternativ können halogenhaltige Lösungsmittel wie Chloroform, Dichlormethan oder Tetrachlorkohlenstoff oder andere heteroatomhaltige Lösungsmittel wie Tetrahydrofuran verwendet werden. Ferner können Carbonsäurederivate wie Ester wie insbesondere Essigsäureethylester oder, Methacrylsäuremethylester, Ketone wie Aceton, Butanon, Amine, wie Ethanolamin, Amiden, wie N,N-Dimethylformamid; Sulfoxide wie Dimethylsufoxid; Nitrile, wie Acetonitril; Fette und Öle, wie Mandelöl, Distelöl, Walnussöl, Weizenkeimöl, Leinöl, Rhizinusöl Verwendung finden.

Die nachstehende Tabelle 1 gibt eine Übersicht über die Löslichkeit erfindungsgemäß komplexierter DNA in einer Reihe von speziellen Lösungsmitteln. Tabelle 1:

| **Lösungsmittel** | **Löslichkeit komplexierter DNA ist größer als** |
|---|---|
| | **(mg DNA / ml Lösungsmittel)** |
| Methanol | 10 |
| 1-Butanol | 10 |
| 1-Pentandiol | 1 |
| 2-Butanon | 1 |
| 2-Propanol | 10 |
| Chloroform | 1 |
| Cyclohexan | 1 |
| Dichlormethan | 1 |
| Dimethylformamid | 1 |
| Ethanol | 10 |
| Ethylenglykol | 1 |
| Ethylenglykolmonobutylether | 10 |
| Heptan | 1 |
| Propylenglykol | 1 |
| Styrol | 1 |
| Tetrachlorkohlenstoff | 1 |
| Tetrahydrofuran | 1 |
| Xylol | 1 |

Nach Herstellung der komplexierten Nukleinsäure kann diese erfindungsgemäß durch Kontaktierung mit einer Kunststoff enthaltenden Lösung zu dem erfindungsgemäßen Kunststoff verarbeitet werden. Dazu wird im Allgemeinen die wie zuvor beschrieben hergestellte komplexierte Nukleinsäure mit einer Lösung des Kunststoffs kontaktiert und vermischt.

Als Kunststoffe können Homopolymere, d.h. Polymere mit Einheiten, die von nur einem Monomer abgeleitet sind, oder CoPolymere eingesetzt werden, d.h. Kunststoffe die von zwei oder mehreren Monomeren abgeleitete unterschiedliche Einheiten aufweisen. Geeignete Polymere bzw. Copolymere sind beispielsweise Polyolefine mit aliphatischen oder aromatischen Einheiten, Polyalkylene, Polyalkylene mit Ungesättigtheiten, halogensubstituierte Polyalkylene, Polyoxyalkylene, Polystyrol und StyrolCopolymere, Polyester, Polyether, Polyamide, Polycarbonate, Polyurethane, Polysiloxane, Poly(meth)acrylsäure und deren Ester und weitere Derivate wie Polyacrylnitril, Polyvinylmaterialen wie Polyvinylacetate, -alkohole, -halogenide, etc. und Copolymere oder höhere Copolymere mit einer Kombination von Einheiten der vorstehenden Polymere wie Styrol-Acrylnitril-Copolymere oder Acrylnitril-Butadien-Styrol-Terpolymere sowie von natürlichen Polymeren abgeleitete Kunststoffe wie Celluloseester oder -ether. Besonders vorteilhaft ist, dass auch biologisch abbaubare Polymere wie z.B. Polylactide Verwendung finden können.

Die folgende Liste von Kunststoffen in Tabelle 2 nennt erfindungsgemäß geeignete Beispiele, wobei diese Liste nicht einschränkend zu verstehen ist.

Die nachstehende Tabelle 2 gibt ferner beispielhafte Lösungsmittel an, in denen die vorstehend genannten Kunststoffe gelöst und mit erfindungsgemäß komplexierter Nukleinsäure kombiniert werden können.

**Tabelle 2:**

| **Polymer** | **Lösungsmittel** | | | | |
|---|---|---|---|---|---|
| | Dichlormethan | Dimethyl-formamid | Methanol | Tetrahydrofuran | Xylol |
| Acylnitril-Butadien-Styrol | X | X | | X | |
| Celluloseacetat | X | X | | X | X |
| Cellulosetriacetat | X | | | | |
| Cellulosemethylether | X | | | | |
| Polyacrylester | | | | | X |
| Polyacrynitril | | X | | | |
| Polyacrylsäure | | X | X | | |
| Polyacrylsäureester | X | | | X | |
| Polymethacrylsäureester | X | | | X | |
| Polybutadien | X | | X | X | X |
| Polybuten-1 | | | | | X |
| Polycarbonat | X | X | | X | |
| Poly-ε-caprolacton | X | | | | |
| Polydimethylsiloxan | X | | | | |
| Polyester (aliphatisch) | X | | | | |
| Polyethylen | | | | | X |
| Polyethylenoxid | | X | | | |
| Polyformaldehyd | | X | | | |
| Polyisobutylen | | | | | X |
| Polymethacrylat | X | X | | | X |
| Polyoxymethylen | X | X | | | |
| Polypropylen (isotaktisch) | | | | | X |
| Polystyrol | X | X | X | X | X |
| Polytrifluorchlorethylen | | | | | X |
| Polyvinylacetat | X | | X | | |
| Polyvinylalkohol | | X | | | |
| Polyvinylchlorid | | X | | X | |
| Polyvinylfluorid | | X | | | |
| Polyisobutylether | X | | | | |
| Styrol-Acrylnitril-Copolymere | X | X | | X | X |
| Polylactid | | | X | | |

Alternativ kann erfindungsgemäß so vorgegangen werden, dass der Kunststoff polymerisiert wird, während er mit der komplexierten Nukleinsäure kontaktiert wird, wobei gegebenenfalls Lösungsmittel vorhanden ist. Alternativ kann in Abhängigkeit von dem jeweiligen Monomer auch das Monomerengemisch selbst als "Lösungsmittel" für die komplexierten Nukleinsäuren dienen.

Vorteilhaft bei einer Polymerisation in Gegenwart der komplexierten Nukleinsäure ist, dass aus dieser Polymerisation auch Nukleinsäure-haltige Kunststoffe gewonnen werden können, für die keine geeigneten Lösungsmittel existieren. Z.B. können quervernetzte Kunststoffe mit Nukleinsäuren hergestellt werden, die aufgrund ihrer Quervernetzung unlöslich sind.

Die Verwendung von Lösungsmitteln, in denen sowohl die komplexierte Nukleinsäure als auch der interessierende Kunststoff löslich sind, eröffnet eine einfache und kostengünstige Möglichkeit der Herstellung des erfindungsgemäßen Kunststoffs.

Bei der Herstellung von Lösungen von komplexierter Nukleinsäure und Kunststoff kann also zum Beispiel so vorgegangen werden, dass zunächst eine Lösung eines oder mehrer polymerer Kunststoffe in Lösungsmittel hergestellt wird und die komplexierte Nukleinsäure sodann in dieser Lösung ebenfalls gelöst wird. Selbstverständlich kann der Kunststoff auch in Gegenwart der gelösten, komplexierten Nukleinsäure gelöst werden. Daraus kann dann der Kunststoff durch Abtrennung des Lösungsmittels leicht gewonnen werden. Ebenfalls ist es möglich, die Nukleinsäure zunächst in Lösungsmittel zu lösen und sodann den polymeren Kunststoff hinzuzufügen. Ferner ist es möglich, zunächst getrennte Lösungen von sowohl der komplexierten Nukleinsäure als auch des polymeren Kunststoffs herzustellen, wobei dasselbe oder unterschiedliche Lösungsmittel verwendet werden können, und die beiden Lösungen sodann zu mischen und anschließend das Lösungsmittel abzutrennen. Soweit der polymere Kunststoff selbst als Flüssigkeit vorliegt und die komplexierte Nukleinsäure in diesem Polymer löslich ist, kann erfindungsgemäß auch auf die Verwendung eines Lösungsmittels gegebenenfalls vollständig verzichtet werden. In diesem Fall entfällt natürlich auch der Schritt des Abtrennens des Lösungsmittels.

Die Abtrennung des Lösungsmittels erfolgt dann beispielsweise durch Abziehen unter Vakuum oder durch Verfahren wie Aufsprühen auf Träger, auf denen der Kunststoff aufgebracht werden soll (Sprühtrocknung). Derartige Verfahren sind dem Fachmann an sich bekannt.

Die erfindungsgemäßen Kunststoffe können vorteilhaft zu Folien, Implantaten, Herzklappen, Prothesen und ähnlichen Gegenständen oder Teilen davon verarbeitet werden oder auf diesen als Beschichtungen aufgebracht werden. Z.B. kann ein erfindungsgemäßer Kunststoff mit einer komplexierten DNA, die für Proteine kodiert, als Teil (z.B. als Beschichtung oder als Massenteil) eines Implantats verwendet werden, um ein besseres Anwachsen im Körper zu ermöglichen. Genauso kann ein Ansiedeln von Zellen auf dem Implantat verhindert werden. Z.B. ist eine Ansiedlung von Zellen auf Stents unerwünscht.

Die folgenden Ausführungsbeispiele veranschaulichen weitere bevorzugte Ausführungsformen der vorliegenden Erfindung.

### Beispiele

Soweit nicht anders bezeichnet stammen alle Chemikalien von der Firma Sigma Aldrich Chemie GmbH, Eschenstrasse 5, 82024 Taufkirchen, Deutschland.

### Beispiel 1

### Herstellung von fluoreszierender, komplexierter Nukleinsäure in Butanol

10 mg Heringssperma DNA wurden in 1 ml demineralisiertem Wasser gelöst. Zu der Lösung wurden 10 µl einer 1 mM fluoreszierender einer synthetischen FAM-DNA (Sequenz-Nr. 1, Hersteller Firma Biolegio B.V., PO BOX 91, 6500 AB Nijmegen, Niederlande) in Wasser gegeben. Zu der entstandenen Lösung wurde ein gleiches Volumen 100 mM wässriger Lösung von Cetyltrimethylammoniumbromid gegeben und die Flüssigkeit gemischt. Es entstand ein gelber Niederschlag, der durch 5 min Zentrifugation bei 15.000 Upm abzentrifugiert wurde. Die flüssige Oberphase wurde verworfen und der Niederschlag in 1ml demineralisiertem Wasser suspendiert und erneut wie oben zentrifugiert. Die wässrige Phase wurde verworfen und der Niederschlag über Nacht im Vakuum getrocknet. Der getrocknete Niederschlag wurde in 1 ml Butanol gelöst.

### Beispiel 2

### Herstellung von fluoreszierender, komplexierter Nukleinsäure in Ethylenglycolmonoethylether

10mg Heringssperma DNA wurden in 1 ml demineralisiertem Wasser gelöst. Zu der Lösung wurden 10 µl einer 1mM fluoreszierender einer synthetischen FAM-DNA (Sequenz-Nr. 1, Hersteller Firma Biolegio B.V., PO BOX 91, 6500 AB Nijmegen, Niederlande) in Wasser gegeben. Zu der entstandenen Lösung wurde ein gleiches Volumen 100 mM wässriger Lösung von Cetyltrimethylammoniumbromid gegeben und die Flüssigkeit gemischt. Es entstand ein gelber Niederschlag, der durch 5 min. Zentrifugation bei 15.000 Upm abzentrifugiert wurde. Die flüssige Oberphase wurde verworfen und der Niederschlag in 1ml demineralisierten Wasser suspendiert und erneut wie oben zentrifugiert. Die wässriger Phase wurde verworfen und der Niederschlag über Nacht im Vakuum getrocknet. Der getrocknete Niederschlag wurde in 1 ml Ethylenglycolmonoethylether gelöst.

### Beispiel 3

### Herstellung von Polystyrol-Lösung mit komplexierter, fluoreszierender DNA

4 g Polystyrol (B + S Chemie GmbH, Poststraße 49, D - 52477 Alsdorf, Deutschland) wurden in 40 ml THF gelöst und so eine 10 %ige (w/v) Polystyrol-Lösung hergestellt. 900 µl der Polystyrol-Lösung wurden mit 100µl der gemäß Beispiel 1 hergestellten FAM-DNA-Lösung in Butanol gemischt. Es bildete sich eine klare Lösung. Die Polystyrol-Lösung mit komplexierter, fluoreszierender DNA wurde 5 min bei 15.000 Upm zentrifugiert. Es bildete sich kein Pellet.

### Beispiel 4

### Herstellung von Polystyrol-Kunststoff mit komplexierter, fluoreszierender DNA durch Ausfällen in Wasser

100 µl der gemäß Beispiel 3 erzeugten Polystyrol-Lösung mit komplexierter, fluoreszierender DNA wurden in einer Kunststoff-Pipette aufgenommen und schnell in ein Gefäß mit 10ml demineralierten Wasser pipettiert. Es entstand ein heller, feiner Niederschlag. Der Niederschlag wurde durch Zentrifugation (5 min, 1.000 Upm) abzentrifugiert und unter dem Fluoreszenzmikroskop ausgewertet. Als Kontrolle wurde eine Polystyrol-Lösung ohne FAM-DNA verwendet. Im Fluoreszenzmikroskop zeigten sich Partikel in einer Größenordnung von 1 bis 10 µm und kleiner. Die Partikel, die aus der Polystyrol-Lösung mit FAM-DNA entstanden, zeigten eine deutliche und homogene, grüne Fluoreszenz. Zur Kontrolle, ob die Nukleinsäuren im Inneren der Partikel oder nur auf der Oberfläche angeordnet sind, wurden die Partikel in 1 ml Ethanol suspendiert und wieder durch Zentrifugation sedimentiert. Im Fluoreszenzmikroskop zeigte sich keine Abnahme der Fluoreszenz gegenüber einer nicht mit Ethanol gewaschen Probe.

### Beispiel 5

### Herstellung von Polystyrol-Kunststoff mit komplexierter, fluoreszierender DNA durch Versprühen an Luft

500 µl der gemäß Beispiel 3 erzeugten Polystyrol-Lösung mit komplexierter, fluoreszierender DNA wurde in eine Airbrush-Pistole gegeben unter einem Abzug auf Glasobjektträger gesprüht. Die niedergeschlagenen Partikel wurden im Fluoreszenzmikroskop ausgewertet. Als Kontrolle wurde eine Polystyrol-Lösung ohne FAM-DNA verwendet. Im Fluoreszenzmikroskop zeigten sich Partikel in einer Größenordnung von 1 bis 10 µm und kleiner. Die Partikel, die aus der Polystyrol-Lösung mit FAM-DNA entstanden wurden, zeigten eine deutliche und homogene, grüne Fluoreszenz.

### Beispiel 6

### Herstellung von Polycarbonat-Lösung mit komplexierter, fluoreszierender DNA

2 g Polycarbonat (B + S Chemie GmbH, Poststraße 49, D - 52477 Alsdorf, Deutschland) wurden in 20ml THF gelöst und so eine 10 %ige (w/v) Polycarbonat-Lösung hergestellt. 900 µl der Polycarbonat-Lösung wurden mit 100 µl der unter Beispiel 1 hergestellten FAM-DNA-Lösung in Butanol gemischt. Es bildete sich eine klare Lösung. Die Polycarbonat-Lösung mit komplexierter, fluoreszierender DNA wurde 5 min bei 15.000 Upm zentrifugiert. Es bildete sich kein Pellet.

### Beispiel 7

### Herstellung von Polycarbonat-Kunststoff mit komplexierter, fluoreszierender DNA durch Ausfällen in Wasser

100 µl der gemäß Beispiel 7 erzeugten Polycarbonat-Lösung mit komplexierter, fluoreszierender DNA wurde in einer Kunststoff-Pipette aufgenommen und schnell in ein Gefäß mit 10 ml demineralierten Wasser pipettiert. Es entstand ein heller, feiner Niederschlag. Der Niederschlag wurde durch Zentrifugation (5 min, 1.000 Upm) abzentrifugiert und unter dem Fluoreszenzmikroskop ausgewertet. Als Kontrolle wurde eine Polycarbonat-Lösung ohne FAM-DNA verwendet. Im Fluoreszenzmikroskop zeigten sich Partikel in einer Größenordnung von 1 bis 10 µm. Die Partikel, die aus der Polycarbonat-Lösung mit FAM-DNA entstanden wurden, zeigten eine deutliche und homogene, grüne Fluoreszenz.

### Beispiel 8

### Herstellung von Acrylnitril-Butadien-Styrol-Kunststoff mit komplexierter, fluoreszierender DNA

1 g Acrylnitril-Butadien-Styrol Polymer (B + S Chemie GmbH, Poststraße 49, D - 52477 Alsdorf, Deutschland) wurde in 50 ml N,N-Dimethylformamid und bzw. THF gelöst, so dass eine 2 %ige (w/v) Acrylnitril-Butadien-Styrol-Polymer-Lösung entstand. Zu je 90 µl der Acrylnitril-Butadien-Styrol-Polymer-Lösungen wurden 10 µl der in Beispiel 2 hergestellten Ethylenglycolmonoethylether-Lösung mit fluoreszierender Nukleinsäure gegeben und gemischt. Zu je 100 µl der hergestellten Lösungen wurden in einer Kunststoff-Pipette aufgenommen und schnell in ein Gefäß mit 10 ml demineraliertem Wasser pipettiert. Es entstand ein Niederschlag. Die Niederschläge wurden durch Zentrifugation (5 min, 1.000 Upm) abzentrifugiert und unter dem Fluoreszenzmikroskop ausgewertet. Als Kontrolle wurden Acrylnitril-Butadien-Styrol-Polymer-Lösungen in THF und DMF ohne FAM-DNA verwendet. Im Fluoreszenzmikroskop zeigten sich faserige Strukturen. Die faserigen Strukturen, die aus dem Acrylnitril-Butadien-Styrol-Polymer-Lösungen mit FAM-DNA entstanden, zeigten eine deutliche und homogene, grüne Fluoreszenz.

### Beispiel 9

### Herstellung von Styrol-Acrylnitril -Kunststoff mit komplexierter, fluoreszierender DNA

1 g Styrol-Acrylnitril-Polymer (B + S Chemie GmbH, Poststraße 49, D - 52477 Alsdorf, Deutschland) wurde in 50 ml N,N-Dimethylformamid und bzw. THF gelöst, so dass eine 2 %ige (w/v) Styrol-Acrylnitril-Polymer-Lösung entstand. Zu je 90 µl der Styrol-Acrylnitril-Polymer-Lösungen wurden 10 µl der in Beispiel 2 hergestellten Ethylenglycolmonoethylether-Lösung mit fluoreszierender Nukleinsäure gegeben und gemischt. Zu je 100 µl der hergestellten Lösungen wurden in einer Kunststoff-Pipette aufgenommen und schnell in ein Gefäß mit 10ml demineraliertem Wasser pipettiert. Es entstanden feine Niederschläge. Die Niederschläge wurden durch Zentrifugation (5 min, 1.000 Upm) abzentrifugiert und unter dem Fluoreszenzmikroskop ausgewertet. Als Kontrolle wurden Styrol-Acrylnitril-Polymer-Lösungen in THF und DMF ohne FAM-DNA verwendet. Im Fluoreszenzmikroskop zeigten sich faserige Strukturen. Die faserigen Strukturen, die aus der Styrol-Acrylnitril-Polymer-Lösungen mit FAM;-DNA entstanden wurden, zeigten eine deutliche und homogene, grüne Fluoreszenz.

### Beispiel 10

### Herstellung von PMMA-Kunststoffen mit komplexierter, fluoreszierender DNA durch Polymerisation

495 µl Methacrylsäuremethylester mit 5 µl der gemäß Beispiel 2 hergestellten Ethylenglycolmonoethylether-Lösung mit fluoreszierender Nukleinsäure gegeben und die Flüssigkeit gemischt. Als Polymerisationsstarter wurden der Flüssigkeit ca. 5 mg Dibenzoylperoxid zugefügt. Ca. 50 ml wurden auf einen Objektträger gegeben. Zum Start der Polymerisation wurde der Objektträger auf einer Wärmeplatte für 10 min auf 60 °C erhitzt. Das entstanden Polymer wurde unter dem Fluoreszenzmikroskop ausgewertet. Als Kontrolle wurde eine Polymerisation ohne FAM-DNA verwendet. Im Fluoreszenzmikroskop zeigte eine grüne Fluoreszenz in den Ansätzen mit FAM-DNA.

### Beispiel 11

### Herstellung von Polystyrol-Kunststoffen mit komplexierter, fluoreszierender DNA durch Polymerisation

450 µl Styrol mit 50 µl der gemäß Beispiel 2 hergestellten Ethylenglycolmonoethylether-Lösung mit fluoreszierender Nukleinsäure gegeben und die Flüssigkeit gemischt. Als Polymerisationsstarter wurden der Flüssigkeit ca. 5 mg Dibenzoylperoxid zugefügt. Ca. 5 0ml wurden auf einen Objektträger gegeben. Zum Start der Polymerisation wurde der Objektträger auf einer Wärmeplatte für 60 min auf 100 °C erhitzt. Das entstanden Polymer wurde unter dem Fluoreszenzmikroskop ausgewertet. Als Kontrolle wurde eine Polymerisation ohne FAM-DNA verwendet. Im Fluoreszenzmikroskop zeigte eine grüne Fluoreszenz in den Ansätzen mit FAM-DNA.

### Beispiel 12

### Herstellung eines RNA-haltigen, biologisch abbaubaren Kunststoffs

10mg Polylactid-Polymer (Aldi-Kunststofftüte) wurde in 1ml THF durch Rühren über Nacht gelöst. 10mg Hefe-RNA in 1ml Wasser wurde durch Zugabe von 1ml 100mM CTAB in Wasser ausgefällt, zentrifugiert, der Niederschlag getrocknet und in 1ml Methanol aufgenommen. 100µl der in Methanol gelösten RNA wurde zu lml der 1% (w/v) Polylactid-Lösung in THF gegeben und gemischt. Zu der Mischung wurde 900µl Wasser zugefügt. Es entstand ein festes glasiges Pellet, das die Nukleinsäure enthielt.

### Beispiel 13

### Ermittlung der Mindestlöslichkeit von komplexierter Nukleinsäure in verschiedenen Lösungsmitteln

1 g Heringssperma-DNA wurden in 100 ml demineralisiertem Wasser gelöst. Je 0,5 ml wurden in Kunststoffreaktionröhrchen überführt. In jedes Röhrchen wurde 0,5 ml wässrige 100mM CTAB-Lösung zugefügt. Die Röhrchen wurden gevortex. Es entstand ein weisser Niederschlag, der durch Zentrifugation für 5 min bei 10.000 xg sedimentiert wurde. Der Überstand wurde verworfen, der Niederschlag in Wasser suspendiert und erneut zentrifugiert. Der Überstand wurde erneut verworfen und der Niederschlag über Nacht unter Vakuum getrocknet.

Je 0,5 ml der folgenden Lösungsmittel: Methanol, Ethanol, Butanol, Ethylenglykolmonobutylether, Propanol wurden in jeweils ein Röhrchen gegeben und mit dem Niederschlag bei Raumtemparatur geschüttelt. Der Niederschlag wurde durch die Lösungsmittel gelöst. Um zu prüfen, ob die komplexierte Nukleinsäure als Suspension vorliegt, wurden die Röhrchen für 5min bei 10.000xg zentrifugiert. Nach der Zentrifugation war kein Niederschlag sichtbar.

Je 100 µl der komplexierten DNA in Butanol und Ethylenglykolmonobutylether (10 mg/ml) wurden zu je 900 µl der folgenden Lösungsmittel: Pentandiol, Butanon, Chloroform, Cyclohexan, Dichlormethan, Dimethylformamid, Ethylenglycol, Propylenglycol, Styrol, Tetrachlorkohlenstoff, Tetrahydrofuran, Xylol gegeben und gemischt. Es entstanden klare Löungen. Um zu prüfen, ob die komplexierte Nukleinsäure als Suspension vorliegt, wurden die Röhrchen für 5 min bei 10.000 xg zentrifugiert. Nach der Zentrifugation war kein Niederschlag sichtbar.

## Patentansprüche

1. Kunststoff enthaltend mindestens eine Nukleinsäure, **dadurch gekennzeichnet, dass** die mindestens eine Nukleinsäure derart komplexiert ist, dass die negativen Ladungen der Nukleinsäure durch organische Kationen im Wesentlichen vollständig kompensiert sind, wobei die organischen Kationen quartäre Ammoniumionen umfassen und jedes organische Kation eine kationische Gruppe trägt.

2. Kunststoff nach Anspruch 1, **dadurch gekennzeichnet, dass** der Kunststoff im Wesentlichen keine organischen Kationen enthält, die über die zur Kompensation der negativen Ladungen der Nukleinsäure erforderliche Menge hinaus gehen.

3. Kunststoff nach Anspruch 1, **dadurch gekennzeichnet, dass** das Molverhältnis zwischen den Anionen der Nukleinsäure und den Ladungen der organischen Kationen im Wesentlichen 1 : 1 beträgt.

4. Kunststoff nach Anspruch 1, **dadurch gekennzeichnet, dass** die Anzahl der gesamten organischen Kationen mit der Anzahl der negativen Ladungen der Nukleinsäure im Wesentlichen identisch ist.

5. Kunststoff nach einem der vorhergehenden Ansprüche, **dadurch gekennzeichnet, dass** die organischen Kationen aus einem Lipid stammen.

6. Kunststoff nach einem der vorhergehenden Ansprüche, **dadurch gekennzeichnet, dass** die organischen Kationen aus CTAB stammen.

7. Kunststoff nach einem der vorhergehenden Ansprüche, **dadurch gekennzeichnet, dass** die mindestens eine komplexierte Nukleinsäure ausgewählt ist aus der Gruppe bestehend aus in komplexierter Form in Wasser unlöslichen Nukleinsäuren, in komplexierter Form in einem von Methanol, Ethanol, Propanol, Butanol, Pentanol, Hexanol oder EGE löslichen Nukleinsäuren und in komplexierter Form in Mischungen eines Alkohols mit einem von DMF, DCM, THF, Heptan, Xylol, Chloroform oder Tetrachlorkohlenstoff löslichen Nukleinsäuren.

8. Kunststoff nach Anspruch 7, wobei die Löslichkeit der komplexierten Nukleinsäure in den in Anspruch 7 genannten Lösungsmitteln oder Mischungen größer ist als 1 mg/ml.

9. Kunststoff nach einem der vorhergehenden Ansprüche, **dadurch gekennzeichnet, dass** die Konzentration der komplexierten Nukleinsäure in dem Kunststoff größer als 0,01 Gew.-% ist, vorzugsweise größer als 0,1 Gew.-%, insbesondere größer als 1 Gew.-%.

10. Kunststoff nach einem der vorhergehenden Ansprüche, **dadurch gekennzeichnet, dass** die mindestens eine Nukleinsäure ausgewählt ist aus der Gruppe bestehend aus synthetischen Nukleinsäuren, natürlichen Nukleinsäuren, Nukleinsäuren, die mindestens ein Gen enthalten, einzelsträngigen Nukleinsäuren, doppelsträngigen Nukleinsäuren, Nukleinsäuren mit mindestens einer nicht natürlich vorkommenden Modifikation.

11. Kunststoff nach einem der vorhergehenden Ansprüche, **dadurch gekennzeichnet, dass** der Kunststoff ausgewählt ist aus der Gruppe bestehend aus Polystyrol, Polycarbonat, SAN-Polymeren (Styrol-Acrylnitril-Copolymere), Acryl-Butadien-Styrol-Copolymer, Polymethylmethacrylat, Polylactiden, biologisch abbaubarem Kunststoff, Kunststoff mit Estergruppen, in organischem Lösungsmittel löslichem Kunststoff, in Wasser unlöslichem Kunststoff.

12. Kunststoff nach Anspruch 11, **dadurch gekennzeichnet, dass** der Kunststoff in einem Lösungsmittel ausgewählt aus der Gruppe bestehend aus DMF, DCM, THF, Heptan, Xylol, Chloroform, Tetrachlorkohlenstoff und Mischungen derselben mit einem oder mehreren Alkoholen löslich ist.

13. Kunststoff nach einem der Ansprüche 1 bis 11, wobei der Kunststoff z. B. aufgrund von Quervernetzungen in Lösungsmitteln unlöslich ist.

14. Kunststoff nach einem der vorhergehenden Ansprüche, **dadurch gekennzeichnet, dass** der Kunststoff als Mikropartikel, Nanopartikel, Pellets, Fasern oder Folie vorliegt.

15. Kunststoff nach einem der vorhergehenden Ansprüche, **dadurch gekennzeichnet, dass** der Kunststoff auf ein humanmedizinisches oder veterinär-medizinisches Implantat aufgebracht ist.

16. Kunststoff nach einem der vorhergehenden Ansprüche, **dadurch gekennzeichnet, dass** der Kunststoff als humanmedizinisches oder veterinär-medizinisches Implantat geformt ist.

17. Verfahren zur Herstellung eines polymeren Kunststoffs gemäß einem der vorhergehenden Ansprüche, bei dem
a) zumindest die folgenden Bestandteile:
i) mindestens eine komplexierte Nukleinsäure, deren negative Ladungen durch organische Kationen im Wesentlichen vollständig komplexiert sind,
ii) mindestens ein polymerer Kunststoff oder ein oder mehrere verschiedene Monomere, aus denen der polymere Kunststoff gebildet wird,
iii) gegebenenfalls organisches Lösungsmittel, in dem die komplexierte Nukleinsäure und der polymere Kunststoff löslich sind, soweit die Monomere nicht bereits ein Lösungsmittel für die komplexierte Nukleinsäure darstellen,
gleichzeitig oder nacheinander zu einer Flüssigkeit vermischt werden,
b) das gegebenenfalls vorhandene Lösungsmittel anschließend abgetrennt wird bzw. die Polymerisation der Monomere durchgeführt wird, wodurch ein Kunststoff entsteht, in dem die negativen Ladungen der Nukleinsäure durch organische Kationen im Wesentlichen vollständig komplexiert sind.

18. Verfahren zur Herstellung eines polymeren Kunststoffs nach Anspruch 17, bei dem als Bestandteil (i) mindestens ein polymerer Kunststoff verwendet wird und organisches Lösungsmittel (iii) in Stufe (a) vorhanden ist.

19. Verfahren zur Herstellung eines Kunststoffs nach Anspruch 17, bei dem als Bestandteil (i) ein oder mehrere verschiedene Monomere eingesetzt werden, aus denen der polymere Kunststoff gebildet wird.

20. Verfahren zur Herstellung eines Kunststoffs nach Anspruch 17, bei dem als Bestandteil (i) ein oder mehrere verschiedene Monomere, aus denen der polymere Kunststoff gebildet wird, eingesetzt werden und diese gleichzeitig ein Lösungsmittel für die komplexierte Nukleinsäure darstellen, so dass kein gesondertes Lösungsmittel (iii) verwendet wird.

## Claims

1. Plastic containing at least one nucleic acid, **characterized in that** the at least one nucleic acid is complexed in such a manner that the negative charges of the nucleic acid are substantially completely compensated for by organic cations, wherein the organic cations comprise quaternary ammonium ions and each organic cation bears a cationic group.

2. Plastic according to Claim 1, **characterized in that** the plastic contains substantially no organic cations which go beyond the amount required for compensation of the negative charges of the nucleic acid.

3. Plastic according to Claim 1, **characterized in that** the molar ratio between the anions of the nucleic acid and the charges of the organic cations is substantially 1:1.

4. Plastic according to Claim 1, **characterized in that** the number of the total organic cations is substantially identical to the number of the negative charges of the nucleic acid.

5. Plastic according to any one of the preceding claims, **characterized in that** the organic cations originate from a lipid.

6. Plastic according to any one of the preceding claims, **characterized in that** the organic cations originate from CTAB.

7. Plastic according to any one of the preceding claims, **characterized in that** the at least one complexed nucleic acid is selected from the group consisting of nucleic acids that are water-insoluble in complexed form, nucleic acids that are soluble in complexed form in one of methanol, ethanol, propanol, butanol, pentanol, hexanol or EGE, and nucleic acids that are soluble in complexed form in mixtures of an alcohol with one of DMF, DCM, THF, heptane, xylene, chloroform or carbon tetrachloride.

8. Plastic according to Claim 7, wherein the solubility of the complexed nucleic acid in the solvents or mixtures cited in Claim 7 is greater than 1 mg/ml.

9. Plastic according to any one of the preceding claims, **characterized in that** the concentration of the complexed nucleic acid in the plastic is greater than 0.01% by weight, preferably greater than 0.1% by weight, in particular greater than 1% by weight.

10. Plastic according to any one of the preceding claims, **characterized in that** the at least one nucleic acid is selected from the group consisting of synthetic nucleic acids, natural nucleic acids, nucleic acids which contain at least one gene, single-stranded nucleic acids, double-stranded nucleic acids, nucleic acids having at least one modification that does not occur naturally.

11. Plastic according to any one of the preceding claims, **characterized in that** the plastic is selected from the group consisting of polystyrene, polycarbonate, SAN polymers (styrene-acrylonitrile-copolymers), acrylic-butadiene-styrene-copolymer, polymethyl methacrylate, polylactides, biodegradable plastic, plastic with ester groups, plastic soluble in organic solvent, water-insoluble plastic.

12. Plastic according to Claim 11, **characterized in that** the plastic is soluble in a solvent selected from the group consisting of DMF, DCM, THF, heptane, xylene, chloroform, carbon tetrachloride and mixtures of the same with one or more alcohols.

13. Plastic according to any one of Claims 1 to 11, wherein the plastic is insoluble in solvents e.g. owing to crosslinks.

14. Plastic according to any one of the preceding claims, **characterized in that** the plastic is present as microparticles, nanoparticles, pellets, fibres or film.

15. Plastic according to any one of the preceding claims, **characterized in that** the plastic is applied to a human medical or veterinary medical implant.

16. Plastic according to any one of the preceding claims, **characterized in that** the plastic is formed as a human medical or veterinary medical implant.

17. Method for production of a polymeric plastic as claimed in any one of the preceding claims, in which
a) at least the following components:
i) at least one complexed nucleic acid, the negative charges of which are substantially completely complexed by organic cations,
ii) at least one polymeric plastic or one or more different monomers from which the polymeric plastic is formed,
iii) optionally organic solvent in which the complexed nucleic acid and the polymeric plastic are soluble if the monomers are not already a solvent for the complexed nucleic acid,
are mixed simultaneously or serially to form a liquid,
b) the optionally present solvent is then separated off or the polymerization of the monomers is carried out, as a result of which a plastic is formed in which the negative charges of the nucleic acid are substantially completely complexed by organic cations.

18. Method for production of a polymeric plastic according to Claim 17, in which, as component (i), at least one polymeric plastic is used and organic solvent (iii) is present in stage (a).

19. Method for production of a plastic according to Claim 17, in which, as component (i), one or more different monomers are used from which the polymeric plastic is formed.

20. Method for production of a plastic according to Claim 17, in which, as component (i), one or more different monomers from which the polymeric plastic is formed are used, and they are at the same time a solvent for the complexed nucleic acid, so that no separate solvent (iii) is used.

## Revendications

1. Plastique contenant au moins un acide nucléique, **caractérisé en ce que** l'au moins un acide nucléique est complexé de telle sorte que les charges négatives de l'acide nucléique sont compensées essentiellement entièrement par des cations organiques, dans lequel les cations organiques comprennent des ions ammonium quaternaires et chaque cation organique porte un groupe cationique.

2. Plastique selon la revendication 1, **caractérisé en ce que** le plastique ne contient essentiellement pas de cations organiques qui vont au-delà de la quantité nécessaire à la compensation des charges négatives de l'acide nucléique.

3. Plastique selon la revendication 1, **caractérisé en ce que** le rapport molaire entre les anions de l'acide nucléique et les charges des cations organiques se monte essentiellement à 1/1.

4. Plastique selon la revendication 1, **caractérisé en ce que** le nombre de cations organiques totaux est essentiellement identique au nombre de charges négatives de l'acide nucléique.

5. Plastique selon une des revendications précédentes, **caractérisé en ce que** les cations organiques proviennent d'un lipide.

6. Plastique selon une des revendications précédentes, **caractérisé en ce que** les cations organiques proviennent de CTAB.

7. Plastique selon une des revendications précédentes, **caractérisé en ce que** l'au moins un acide nucléique complexé est sélectionné parmi le groupe constitué d'acides nucléiques insolubles dans l'eau sous forme complexée, d'acides nucléiques solubles dans un parmi le méthanol, éthanol, propanol, butanol, pentanol, hexanol ou EGE sous forme complexée et d'acides nucléiques solubles dans des mélanges d'un alcool avec un parmi DMF, DCM, THF, heptane, xylène, chloroforme ou tétrachlorométhane sous forme complexée.

8. Plastique selon la revendication 7, dans lequel la solubilité de l'acide nucléique complexé dans les solvants ou mélanges cités à la revendication 7 est supérieure à 1 mg/ml.

9. Plastique selon une des revendications précédentes, **caractérisé en ce que** la concentration de l'acide nucléique complexé dans le plastique est supérieure à 0,01 % en poids, de préférence supérieure à 0,1 % en poids, notamment supérieure à 1 % en poids.

10. Plastique selon une des revendications précédentes, **caractérisé en ce que** l'au moins un acide nucléique est sélectionné parmi le groupe constitué d'acides nucléiques synthétiques, d'acides nucléiques naturels, d'acides nucléiques qui contiennent au moins un gène, d'acides nucléiques à brin unique, d'acides nucléiques à double brin, d'acides nucléiques avec au moins une modification ne survenant pas naturellement.

11. Plastique selon une des revendications précédentes, **caractérisé en ce que** le plastique est sélectionné parmi le groupe constitué du polystyrène, du polycarbonate, de polymères de SAN (copolymères de styrène-acrylonitrile), d'un copolymère d'acryle-butadiène-styrène, du polyméthylméthacrylate, de polylactides, d'un plastique biologiquement dégradable, d'un plastique avec des groupes ester, d'un plastique soluble dans un solvant organique, d'un plastique insoluble dans l'eau.

12. Plastique selon la revendication 11, **caractérisé en ce que** le plastique est soluble dans un solvant sélectionné parmi le groupe constitué de DMF, DCM, THF, heptane, xylène, chloroforme, tétrachlorométhane et de mélanges de ceux-ci avec un ou plusieurs alcools.

13. Plastique selon une des revendications 1 à 11, dans lequel le plastique est insoluble dans des solvants par exemple en raison de réticulations croisées.

14. Plastique selon une des revendications précédentes, **caractérisé en ce que** le plastique est présent en tant que microparticules, nanoparticules, granulés, fibres ou film.

15. Plastique selon une des revendications précédentes, **caractérisé en ce que** le plastique est appliqué sur un implant de médecine humaine ou de médecine vétérinaire.

16. Plastique selon une des revendications précédentes, **caractérisé en ce que** le plastique est moulé en tant qu'implant de médecine humaine ou de médecine vétérinaire.

17. Procédé de fabrication d'un plastique polymère selon une des revendications précédentes, dans lequel
a) au moins les constituants suivants:
i) au moins un acide nucléique complexé dont les charges négatives sont complexées essentiellement entièrement par des cations organiques,
ii) au moins un plastique polymère ou un monomère ou plusieurs monomères différents à partir desquels le plastique polymère est formé,
iii) éventuellement un solvant organique dans lequel l'acide nucléique complexé et le plastique polymère sont solubles dans la mesure où les monomères ne représentent pas déjà un solvant pour l'acide nucléique complexé,
sont mélangés simultanément ou successivement à un liquide,
b) le solvant éventuellement présent est ensuite séparé ou la polymérisation des monomères est effectuée, moyennant quoi un plastique est produit, dans lequel les charges négatives de l'acide nucléique sont complexées essentiellement entièrement par des cations organiques.

18. Procédé de fabrication d'un plastique polymère selon la revendication 17, dans lequel au moins un plastique polymère est utilisé en tant que constituant (i) et un solvant organique (iii) est présent à l'étape (a).

19. Procédé de fabrication d'un plastique selon la revendication 17, dans lequel un monomère ou plusieurs monomères différents à partir desquels le plastique polymère est formé sont utilisés en tant que constituant (i).

20. Procédé de fabrication d'un plastique selon la revendication 17, dans lequel un monomère ou plusieurs monomères différents à partir desquels le plastique polymère est formé sont utilisés en tant que constituant (i) et ceux-ci représentent simultanément un solvant pour l'acide nucléique complexé de sorte qu'aucun solvant séparé (iii) n'est utilisé.
